# EUROPEAN PATENT SPECIFICATION

(11) **EP 3 405 487 B1**
(45) Date of publication and mention of the grant of the patent: **10.03.2021**
(21) Application number: 17724460.5
(22) Date of filing: 20.01.2017
(51) Int. Cl.: C07K 16/02, A61K 39/00

(54) **IGY ANTIBODIES FOR THE PREVENTION OF SEA LICE INFESTATION AND INFECTION**
IGY-ANTIKÖRPER ZUR VERHINDERUNG VON FISCHLAUSBEFALL UND INFEKTION
ANTICORPS IGY POUR LA PREVENTION DE L'INFESTATION PAR LES POUX DE MER ET DE L'INFECTION ASSOCIÉE

(30) Priority: 22.01.2016 NO 20160113
(43) Date of publication of application: 28.11.2018
(73) Proprietor: Norifish AS, 5430 Bremnes (NO)
(72) Inventor: MORLANDSTØ, Jan Ove, 5427 Urangsvåg (NO); EVENSEN, Øystein, 0198 Oslo (NO); SKJERVOLD, Per Olav, 1430 Ås (NO)
(74) Representative: Acapo AS
(86) International application number: PCT/NO2017/050019
(87) International publication number: WO 2017/126976

(56) References cited:
- WO-A2-2007/146359
- LING-LIN FU ET AL: "Protection of Fenneropenaeus chinensis (Osbeck, 1765) against the white spot syndrome virus using specific chicken egg yolk immunoglobulins by oral delivery : Protection of shrimp against WSSV by specific IgYs", AQUACULTURE RESEARCH, vol. 41, no. 12, 2 November 2010 (2010-11-02), pages 1806-1816, XP055383695, GB ISSN: 1355-557X, DOI: 10.1111/j.1365-2109.2010.02558.x
- LIU ZHENXING ET AL: "Oral Passive Immunization of Carp Cyprinus carpio with Anti-CyHV-3 Chicken Egg Yolk Immunoglobulin (IgY)", FISH PATHOLOGY, vol. 49, no. 3, September 2014 (2014-09), pages 113-120, XP002771247, ISSN: 0388-788X
- D.K KIM ET AL: "Shrimp protected from WSSV disease by treatment with egg yolk antibodies (IgY) against a truncated fusion protein derived from WSSV", AQUACULTURE, vol. 237, no. 1-4, 1 August 2004 (2004-08-01), pages 21-30, XP055383708, Amsterdam, NL ISSN: 0044-8486, DOI: 10.1016/j.aquaculture.2004.03.015
- LU Y ET AL: "Passive protection of shrimp against white spot syndrome virus (WSSV) using specific antibody from egg yolk of chickens immunized with inactivated virus or a WSSV-DNA vaccine", FISH AND SHELLFISH IMMUNOLOGY, ACADEMIC PRESS, LONDON, GB, vol. 25, no. 5, 1 November 2008 (2008-11-01), pages 604-610, XP025609078, ISSN: 1050-4648, DOI: 10.1016/J.FSI.2008.08.010 [retrieved on 2008-09-04]
- YONGPING XU ET AL: "Application of chicken egg yolk immunoglobulins in the control of terrestrial and aquatic animal diseases: A review", BIOTECHNOLOGY ADVANCES, ELSEVIER PUBLISHING, BARKING, GB, vol. 29, no. 6, 11 July 2011 (2011-07-11), pages 860-868, XP028306419, ISSN: 0734-9750, DOI: 10.1016/J.BIOTECHADV.2011.07.003 [retrieved on 2011-07-20]
- RAYNARD ROBERT S ET AL: "Development of vaccines against sea lice", PEST MANAGEMENT SCIENCE, WILEY & SONS, BOGNOR REGIS; GB, vol. 58, no. 6, 1 June 2002 (2002-06-01), pages 569-575, XP002419573, ISSN: 1526-498X, DOI: 10.1002/PS.474

## Description

### Field of the invention

The present invention relates to an IgY antibody, and the use of such an antibody for the reduction of infestation and/or prevention and/or treatment of infection of a Salmonidae by a parasite of the family *Caligidae.* The IgY antibodies are obtained by immunization of an egg-laying poultry with a Caligidae. The invention also relates to a pharmaceutical composition and a vaccine composition comprising such an IgY antibody.

### Background of the invention

*Lepeophtheirus salmonis* or salmon louse belongs to the family *Caligidae* and is a parasitic copepod in salmonids, particularly Atlantic salmon (*Salmo salar* L.). It is found in the northern hemisphere. *L. salmonis* infection has been a significant problem for the aquaculture industry since the 70's and causes significant economic losses and is also an animal welfare problem. The high prevalence of lice in today cages also serves as an infection pressure on wild salmon in the local environment, which is a strong motivation for the aquaculture industry to control and minimize the infection load. *L. salmonis* has been found in farmed fish in Norway, Faroe Islands, Canada, Scotland, Ireland and Japan.

Pathogenicity of the parasite is related to direct physical damage when the infection pressure is high, and there is indication the parasite can serve as vector for various infectious agents like salmon pancreas disease (PD) virus, infectious salmon anaemia virus and *Vibrio salmonicida.* Identification of the pathogen in the louse or associated with the louse is not the same as being a passive or true vector. In addition, the fish resistance to viral infections is possibly reduced as a result of louse immunomodulation and infection may impact fish growth. The clinical picture depends on the host, the number of lice on the fish as well as the developmental stage of the louse.

Host susceptibility is influenced by factors such as stress, nutritional status, immune status and genetic resistance. The mortality in Atlantic salmon is associated with high infection load (number of parasite) and thus release of components from the parasite itself, combined with stress response in salmon at high infestation (release of endogenous cortisol with subsequent immunosuppression) and inadequate immune response against lice. Secondary infections likely play a role, i.e. skin abrasions caused by the parasite facilitate secondary infections.

Vaccination attempts are several although none have been successful so far. In light of the increasing problems with resistance in sea lice, there is an urgent need for alternative control methods and immune intervention is an attractive one and far more sustainable than the current practices of chemical treatments in open cages with release of active ingredient to the surrounding water and the marine environment.

Parasites have complex life cycles and lice are no exception. Sea lice have a direct life cycle, meaning it requires one host to complete its development. The lice must go through eight stages before reaching sexual maturity and development are temperature dependent, approximately 400 degree days, and require high salinity for optimal. After hatching from eggs, lice will undergo two naupilus stages. This is a free-swimming planktonic stage lasts 5-15 days depending on temperature. Then follows the copepodite stage and this is when the lice find a suitable host and attaches. From this stage and until maturation, immune intervention is possible, but different strategies would have to be employed dependent on development stage of the lice. The copepodites will attach to the skin of the fish using special frontal filaments. Predilection sites are on the fins during the early attachment stages, moving over to the skin behind the dorsal fin. There are two chalimus stages and at this stage it is still attached to the host and feed on the skin mucus, cells and excretory products from/in the skin. These stages will take up mucus, skin and underlying tissue through its mouth part and mechanical interaction with the skin. The two pre-adult stages are motile and they can move from one fish to the other. The last stages are preadult and adult and now the sea lice take up also blood/serum from the host to reach maturity, particularly the female to allow the egg strings to mature.

The approach so far (in the literature) in developing a vaccine against sea lice is in several ways different from those sought for bacterial and viral infections. The vaccine aims to prevent replication of the agent in the host for the two latter categories of pathogens, while for sea lice vaccines the aim is to reduce the number of attached lice to the host and also limit the fecundity/production of off-spring. This can be achieved by inhibiting the lice to reproduce or inhibit reproduction ability lice. A 50% reduction in number of sea lice attached could result in an even more pronounced reduction of the infection pressure to the environment.

There have been several attempts to develop a vaccine against sea lice, but today there are no available vaccines with sufficient protective effects (Carpio *et al.,* 2013, Carpio, Y., Garcia, C., Pons, T., Haussmann, D., Rodriguez-Ramos, T., Basabe, L., Acosta, J., Estrada, M.P. 2013. Akirins in sea lice: first steps towards a deeper understanding. Exp Parasitol 135, 188-199.

Atlantic salmon has been immunized with native antigens from an extract made from whole *L. salmonis* parasites. Specific antibody production was recorded for several antigens, but antibodies were not protective when the salmon were then subjected to pre-adult and adult *L. salmonis* infection (Raynard *et al.,* 2002, Raynard, R.S., Bricknell, I.R., Billingsley, P.F., Nisbet, A.J., Vigneau, A., Sommerville, C. 2002. Development of vaccines against sea lice. Pest Manag Sci 58, 569-575).

L-L FU et al., AQUACULTURE RESEARCH, vol. 41, no. 12, pages 1806-1816, XP05S383695, describes the protection of shrimp against white spot syndrome virus infection by IgY specific for WSSV derived from poultry immunized with WSSV antigen.

Liu z et al., FISH PATHOLOGY, vol. 49, no. 3, pages 113-120, describes oral passive immunization of carp with IgY being specific for cyprinid herpes virus 3.

D K KIM et al., AQUACULTURE, vol.237, no. 1-4, pages 21-30, XP055383708, describes protection of shrimp against white spot syndrome virus infection by IgY specific for WSSV.

Lu Y et al., FISH AND SHELLFISH IMMUNOLOGY, vol. 25, no. 5, pages 604-61 0, XP025609078 describes passive protection of shrimp against WSSV using specific IgY of chickens immunized with inactivated virus or a WSSV-DNA vaccine.

Y Xu et al., BIOTECHNOLOGY ADVANCES, vol. 29, no. 6, pages 860-868, XP028306419, describes the application of IgY in the field of disease control in aquatic and terrestrial animals.

WO2007/146359 describes vaccines comprising sea lice antigens used for active vaccination of fish.

Raynard R S et al., PEST MANAGEMENT SCIENCE, vol. 58, no. 6, pages 569-575, XP002419573, describes the development of vaccines against sea lice.

It is thus an object of the invention to prepare and provide an antibody that is effective in the sea lice reproduction and viability in order to reduce the number of sea lice attachment to salmonids after a challenge by sea lice.

The object is accomplished by providing an IgY composition that is prepared by immunizing egg-laying hens with *Lepeophtheirus salmonis* antigens, and the obtained IgY effectively reduces the number of sea lice attached to salmon after challenge.

### Summary of the invention

The present invention provides IgY antibodies that reduced the number of sea lice attached to a salmonid after a sea lice challenge.

A first aspect of the present invention relates to an IgY antibody composition obtained by immunization of an egg-laying poultry with any development stage of a Caligidae sea louse.

Preferably, the Caligidae is a Lepeophtheirus.

Preferably, the Lepeophtheirus is *Lepeophtheirus salmonis.*

Preferably, the Caligidae is a Caligus.

Preferably, the Caligus is Caligus rogercresseyi.

Preferably, the egg-laying poultry is;
a) immunized with a Caligidae,
b) eggs provided by the immunized poultry are collected,
c) preparation of the IgY composition, wherein the egg yolk of the collected eggs is processed, and said IgY is isolated.

Preferably, the Caligidae is *Lepeophtheirus salmonis or Caligus rogercresseyi.*

Preferably, the Caligidae preparation comprises one of the development stages, or a combination of two or more of the development stages, or is a mixture of all the different development stages of said Caligidae.

Preferably, the Caligidae is *Lepeophtheirus salmonis or Caligus rogercresseyi.*

Preferably, the preparation comprises copepodids and chalimus stages.

Preferably, the preparation comprises sea lice in pre-adult and adult stages.

In a second aspect, the present invention relates to a IgY composition for use in reduction of infestation and/or prevention and/or treatment of infection of a Salmonidae by a parasite of the order Caligidae, wherein an IgY composition obtained by immunization of an egg-laying poultry with a Caligidae is administered to said Salmonidae.

Preferably, the Caligidae is a Lepeophtheirus.

Preferably, the Lepeophtheirus is *Lepeophtheirus salmonis.*

Preferably, the Caligidae is a Caligus.

Preferably, the Caligus is Caligus rogercresseyi.

Preferably, the administration route is oral, preferably wherein the IgY composition is administered as a component of the feed given to the Salmonidae.

Preferably, the administration route is by injection, preferably intraperitoneally, preferably as watery solution or any formulation for retaining said IgY at the injection site providing slow release.

Preferably, the Salmonidae is infected by a Caligidae of the genera Lepeophtheirus.

Preferably, the Salmonidae is infected by a Caligidae of the genera Caligus.

Preferably, the Salmonidae is infected by *Lepeophtheirus salmonis.*

Preferably, the Salmonidae is infected by *Caligus rogercresseyi.*

Preferably, said Salmonidae is selected from the group consisting of salmon, charr and trout.

Preferably, said Salmonidae is Atlantic salmon.

Preferably, said Salmonidae is Atlantic salmon smolt.

Preferably, said Salmonidae are any Oncorhynchus species.

A third aspect of the present invention relates to a pharmaceutical composition comprising an IgY antibody composition according to any of the aspects and preferable embodiments above, and at least one medically compatible adjuvant.

A fourth aspect of the present invention relates to a vaccine composition comprising an IgY antibody composition according to any of the aspects and preferable embodiments above.

### Description of the drawings

Fig. 1. Sea lice numbers at 27 and 45 days post infection in different treatment groups. Statistical analysis was performed for IgY versus control fish at each time point and the obtained p-values were p<0.0000 at each time point. Test for normality was performed and confirmed.
Fig. 2. Sea lice numbers at 8, 21 and 32 days post infection (dpi) in different treatment groups. Groups are IgY, IgY-NS, control fish (as indicated).

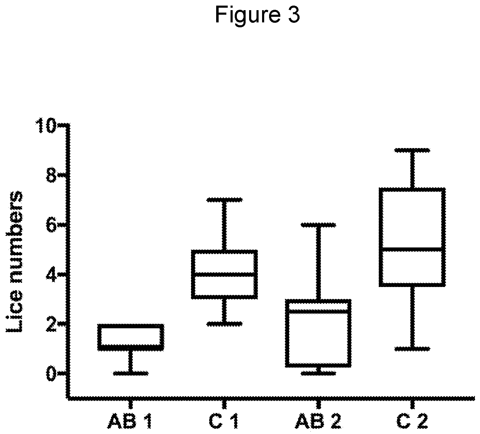

Figure 3 shows that pre-treatment with anti-lice IgY gives a reduction of sea lice at all stages in a cohabitant model.
Figure 4 shows the number of lice two weeks after challenge, as described in example 4. Figure 4a shows the lice number two weeks post challenge, and figure 4b shows lice number 3 weeks post challenge.
Figure 5 shows lice numbers at 1 weeks (figure 5a) and 2 weeks (figure 5b) after injection of soluble IgY (2 weeks after initial challenge with copepodids), as given in example 5. Ctrl is the control group (non-treated) while AB1 and AB3 are as described in the examples 4 and 5. 1w and 2w indicates counting at 1 week or 2 week, respectively, after injection of the water soluble IgY solution (0.05 mg/g live weight). The numbers are given as average ± standard deviation.

### Description of embodiments of the invention

### Experimental section

### Example 1

### Antigen preparation and immunization protocols

Salmon louse, *Lepeophtheirus salmonis,* of different development stages, copepodids/chalimus, pre-adult and adult sea lice with egg-strings, were collected from infected salmon on the west coast of Norway (Hordaland county). The different stages of lice where collected from anesthetized salmon, and transferred to phosphate-buffered formalin (10% w/w) and stored in formalin at 4C until processed.

Antigen preparations were made in two different batches, including batch 1), copepodids and chalimus stages, and batch 2) pre-adult and adult stage (male and female; equal proportions). Approximately 5 lice of each stage were pooled into batch 1) and 2) and homogenized in sterile phosphate buffered saline (PBS), pH=7.2. The batches were pooled, and the homogenates were centrifuged at 2500 x *g* for 5 min (at 4C) and the supernatant was collected (aspirated) using sterile pipettes. The protein concentration was measured using standard methods (Bradford) and adjusted to 20 mg/ml for batch 1 and 40 mg/ml for batch 2.

Chickens (n=6) at egg-laying age (20 weeks old) were injected intramuscularly 4 times, 21 days apart by the antigen preparations.. The first injection was made with the antigen preparation (as described above), mixed 1:1 (weight:weight) with Freund's complete adjuvant and given at a dose of 0.5 ml per chicken (intramuscularly, *Musculus pectoralis majoris*). Subsequent injections were made using Freund's incomplete adjuvant mixed as described. Injection volume was 0.5 ml per chicken. When the hens started egg-laying, extraction of IgY was performed as follows. Twice the egg yolk volume of PBS was mixed with yolk, thereafter 3.5 % polyethylene glycol (PEG) 6000 (in gram) of the total volume was added and vortexed, followed by 10 min rolling on a rolling mixer. The watery phase was collected, ant this watery phase contained the IgY and other proteins. This is followed by centrifugation (3000 rpm) at 4°C, 20 min. The supernatant is poured through a folded filter (0.4 micron) and transferred to a new tube. 8.5 % (w/v) PEG 6000 is added to the tube, the tube is vortexed and rolled on a rolling mixer (max 30 s). This step is repeated and the supernatant is discarded. The pellet is carefully dissolved in 1 ml PBS using the vortexer. PBS is added to a final volume of 10 ml. The solution is mixed with 12 % PEG 6000 (weight/vol, 1.2 gram) and then vortexed.

The pellet was then dissolved carefully in 800 µl PBS (glass stick and vortex) and transferred to a dialysis capsule. The extract was dialysed (holding back >50 kDa size) over night in 0.1 % saline (1,600 ml) and gently stirred using a magnetic stirrer. The next morning, the saline is replaced by PBS and dialysed for another three hours. The IgY-extract was pulled from the dialysis capsule and transferred to 50 ml tubes.

The protein content (mg/mL) of the samples was measured photometrically at 280 nm (1:50 diluted with PBS) and calculated according to the Lambert-Beer law with an extinction coefficient of 1.33 for IgY.

### Immunization of salmon and challenge

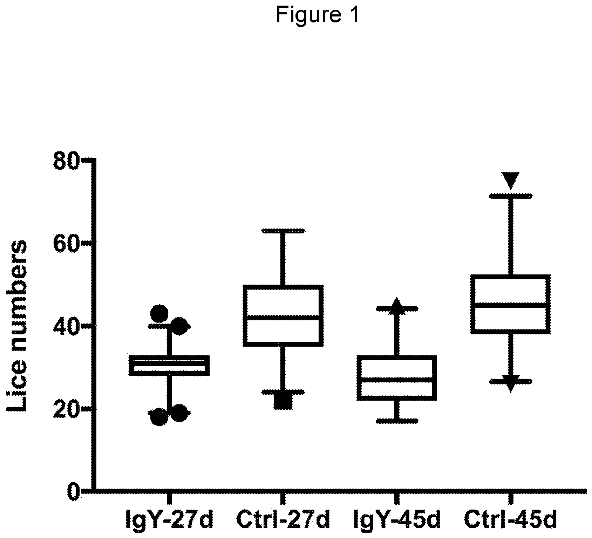

Atlantic salmon (*Salmo salar* L.), AquaGen AS source (obtained from Sør-Smolt, Norway), of 300-350g size, kept in sea water (8 C). The fish were kept in two different tanks (n=34 in each; Fig. 1). The fish were injected with 0.05-0.06 mg IgY/g live weight (1:1 v/v of batch 1 and 2 above), intraperitoneally, using a 24G needle, 1 day prior to challenge with sea lice copepodids. Non-treated controls (n=34 fish per tank) were run in 2 parallel tanks.

90 nauplii of *Lepeophtheirus salmonis,* per fish were added to a tank containing 200L of seawater (32‰). The water flow was turned off during the challenge with the sea lice, and the water was oxygenated through an external source and monitoring continuously using a standard oxygen probe. The concentration of oxygen was not allowed to fall below 8%. The copepodids were allowed to attach to the fish for 30 minutes after which the water flow was resumed and oxygenation ceased.

### Counting of sea lice

Counting of sea lice of different stages was done at 21 days post challenge and then at 42 days post challenge.

At 25 days post challenge, the non-treated controls fish had an average of 45 sea lice (total) per fish while the IgY-treated fish had an average of 29 sea lice/fish; giving a reduction of 35.6% (Fig. 1). At 45 days post challenge, the number of sea lice in the controls was 45/fish while the IgY-treated fish had 28 sea lice/fish, i.e. a 38% reduction (Fig. 1). The experiment was completed at 45 days post challenge and the fish were deloused.

Table 1 shows the statistical comparison between IgY and controls at 27 days post infection. Data were tested for normality by Shapiro-Wilks test (Stata14). Same results were obtained for the 45 days post infection data.

**Table 1**

| Two-sample t test with unequal variances | | | | | | |
|---|---|---|---|---|---|---|
| Variable | Obs | Mean | Std. Err. | Std. Dev. | [95% Conf. | Interval] |
| Ctrl27d | 40 | 42.9 | 1.626227 | 11.28516 | 39.61064 | 46.18936 |
| IgY27d | 40 | 30 | .8704552 | 5.505242 | 28.23934 | 31.76066 |
| combined | 80 | 36.45 | 1.168941 | 10.45533 | 34.12328 | 38.77672 |
| diff | | 12.9 | 1.844535 | | 9.21127 | 16.58873 |
| diff = mean(Ctrl27d) - mean(IgY27d) | | | | | t = 6.9936 | |
| Ho: diff = 0 | | | Welch's degrees of freedom = 60.7112 | | | |
| Ha: diff < 0 | | | Ha: diff != 0 | | Ha: diff > 0 | |
| Pr(T < t) = 1.0000 | | Pr( \|T\| > \|t\|) = 0.0000 | | | Pr(T > t) ) = 0.0000 | |

This experiment clearly shows that the administration of IgY antibodies produces after a challenge with L. salmonis inhibit that infestation and/or attachment of sea lice to the sea lice challenged fish, and can thus be used as a prophylactic and/or curative measure against sea lice infection.

### Example 2

### Antigen preparation and immunization protocols

The sea lice antigen preparation was made as described above and the same immunization protocol was used as described in example 1. This IgY preparation is referred to as IgY. In addition, we have prepared an IgY preparation obtained from immunization and purification protocols as described above, where the antigen was salmon pancreas disease virus (obtained and prepared as described in (Xu *et al.,* 2012). This preparation is referred to as non-specific IgY, (Ig-NS) and was used as a control.

### Immunization of salmon and challenge

Atlantic salmon, 85 g, was obtained from Sør-Smolt (AquaGen AS source). The fish were vaccinated against furunculosis, vibriosis, cold-water vibriosis and winter ulcer. The fish were transported to the experimental facility, and allowed to be acclimatized for 4 weeks prior to transfer to the experimental challenge unit. Fish were transferred to 2 separate tanks, 39 fish per tank. Water level was kept at 400L per tank, 32‰ seawater, 8°C. In tank 1, 19 fish were injected with IgY and 20 fish with IgY-NS. The IgY-NS fish were marked by fin-clipping. In tank 2, 20 fish were injected with IgY and 19 fish with IgY-NS, and this group was fin-clipped. Control fish (non-treated) were kept in 3 parallel tanks, containing 45, 43 and 39 fish per tank.

Counting of sea lice was done as described in Experiment 1, and at 8, 15, 21, and 32 days post challenge. The results are shown in Table 2.

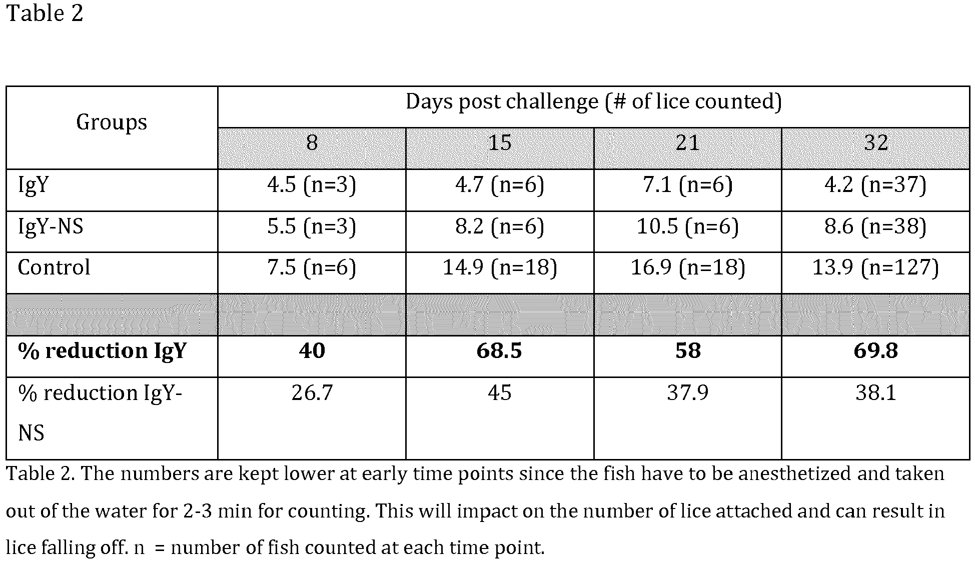

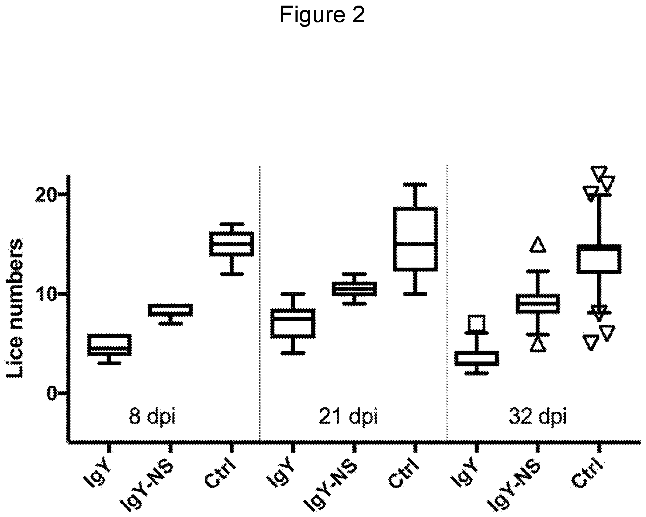

Figure 2 shows the sea lice numbers at 8, 21 and 32 days post infection (dpi) in different treatment groups. Different groups (IgY, IgY-NS and controls) are indicated on the figure.

Table 3 shows the statistical analysis performed for IgY versus control fish (on log-transformed data). Two-sided t-test gives p<0.0000. The same was found for IgY versus IgY-NS groups (not shown). Significant difference was found at all time points.

**Table 3**

| Two-sample t test with equal variances | | | | | | |
|---|---|---|---|---|---|---|
| Variable | 0bs | Mean | Std. Err. | Std. Dev. | [95% Conf. | Interval] |
| logctrl | 60 | 2.60792 | .0330102 | .2556956 | 2.541867 | 2.673973 |
| logIgY | 38 | 1.177097 | .0598753 | .3690959 | 1.055778 | 1.298416 |
| combined | 98 | 2.053111 | .077114 | .7633893 | 1.900061 | 2.206161 |
| diff | | 1.430823 | .0631185 | | 1.305534 | 1.556112 |
| diff = mean(logctrl) - mean(logIgY) | | | | t = 22.6689 | | |
| Ho: diff = 0 | | | | degrees of freedom = 96 | | |
| Ha: diff < 0 | | Ha: diff != 0 | | | Ha: diff > 0 | |
| Pr(T < t) = 1.0000 | | Pr(\|T\| > \|t\|) = 0.0000 | | | Pr(T > t) = 0.0000 | |

In summary, the results show that pre-treatment with anti-lice IgY (0.05 mg/g live weight of fish) gives a highly significant reduction in number of sea lice at all stages from 8 till 32 days post infection. The reduction in sea lice attached to the fish is almost 70% 15 and 32 days after challenging of the fish with sea lice.

The results also show that the IgY-NS antibody (i.e. an IgY antibody with an irrelevant specificity) reduces the number of sea lice attached to the fish. The reduction obtained by administration of the IgY-NS, with regard to sea lice attached to the fish is 38-40% after 15, 21 and 32 days after challenging of the fish with sea lice.

### Example 3

### Antigen preparation and immunization protocols

The sea lice antigen preparation was made as described above and the same immunization protocol was used as described in example 1. This IgY preparation is referred to as IgY.

### Immunization of salmon and challenge

Atlantic salmon, 350 g, were obtained from Sør-Smolt and of AquaGen AS source. The fish were vaccinated against furunculosis, vibriosis, cold-water vibriosis and winter ulcer. The fish had been at the experimental facility since presmolt stage and allowed to acclimatize for 2 weeks prior to transfer to the experimental tanks. Fish were transferred to 2 separate tanks, 15 fish per tank. Water level was kept at approximately 400L per tank, 32%o seawater and at 8-9°C.

In tank 1, 15 fish were injected with IgY, 0.05mg IgY/g live weight. The IgY solution was a 1:1 mix of batch 1 and 2, as described under Example 1. 15 fish were left untreated (no IgY injected). In tank 2, fish (n=30 total) were treated the same way. The day after injection of IgY, 3 large Atlantic salmon (500g) already infected with sea lice (*Lepeophtheirus salmonis*) using the same protocol as described in Exp. 1 and starting from copepodids, were introduced into the tanks. These fish were termed shedder fish and had 50-55 lice average per fish. At 3 weeks after the shedders were introduced, the number of sea lice in the IgY-injected fish and shedder fish were counted.

The lice numbers are given below in tank 1 and 2, respectively. IgY groups are referred to as AB1 (tank 1) and AB2 (tank 2; antibody IgY injected) and C1 and C2 (tank 1 and 2, respectively) is control (no antibody injected). There is a 68 and 65% reduction in the number of lice counted per fish relative to controls. The shedders carried approximately 50 lice per fish (not shown).

The results show that pre-treatment with anti-lice IgY (0.05 mg/g live weight of fish) gives a highly significant reduction in number of sea lice at all stages at 3 weeks post introducing shedder fish into tanks with IgY treated fish, i.e. in a cohabitant model.

### Example 4

### Antigen preparation and immunization protocols

The sea lice antigen preparation was made as described above and the same immunization protocol to obtain anti-lice IgY was used as described in example 1. This IgY preparation is referred to as IgY.

### Immunization of salmon and challenge

Atlantic salmon, average weight 450g, obtained from Sør-Smolt and of AquaGen AS source. The fish were vaccinated against furunculosis, vibriosis, cold-water vibriosis and winter ulcer. The fish had been at the experimental facility since presmolt stage and allowed to acclimatize for 2-3 weeks prior to transfer to the experimental tanks.

Fish were transferred to 2 separate tanks. The IgY preparation was obtained by mixing the watery solution (IgY) with an oil adjuvant (commercially available) yielding a water-in-oil formulation. The final water-in-oil formulation was made by mixing 1:1 of a watery solution of batch 1 and 2 of IgY, as described in Example 1. The watery solution of IgY was then again mixed 1:1 (weight/weight) with the oil adjuvant, and prepared to obtain an emulsion. This IgY formulation was termed AB1. Then a second formulation was made by mixing first a watery solution of batch 1 and 2 of IgY, (cf. Example 1) at 1:3 ratio (batch 1 and 2, respectively) and then mixed 3:7 (weight/weight) with vegetable oil, and prepared to obtain an emulsion. This formulation was termed AB3. 15 fish were then injected intraperitoneally with 0.025mg/g live weight of IgY (final dose) with AB1 and 15 fish with AB3. 15 fish were left untreated, giving a total of 45 fish.

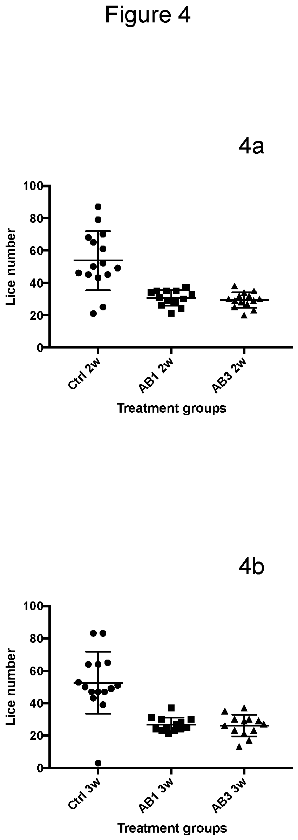

70 days (10 weeks) after the initial injection of AB1 and AB3, all fish were challenged with newly prepared copepodids (70 / fish) using the same protocol as described in Example 1. The number of sea lice counted in average on the fish is given in figure 4. There is a highly significant reduction in number of sea lice per fish following both AB1 and AB3 treatment. The number of sea lice was reduced by 43% and 45% for the AB1 group at 2 (figure 4a) and 3 weeks (figure 4b) post challenge, respectively, and 48% and 49% for the AB3 group at the same time points.

Statistical comparison in based on one-way Anova with Dunnet's post-hoc test giving p-values of 0.0001 for the 2 treatments versus control at both time points.

**2 weeks post challenge:**

| Dunnett's multiple comparisons test | Mean Diff. | 95.00% CI of diff. | Significant? | Summary | Adjusted P Value | A-? | |
|---|---|---|---|---|---|---|---|
| | | | | | | | |
| Ctrl 2w vs. AB1 2w | 23.12 | 13.04 to 33.2 | Yes | **** | 0.0001 | B | AB1 2w |
| Ctrl 2w vs. AB3 2w | 24.3 | 14.42 to 34.19 | Yes | **** | 0.0001 | C | AB3 2w |

**3 weeks post challenge:**

| Dunnett's multiple comparisons test | Mean Diff. | 95.00% CI of diff. | Significant? | Summary | Adjusted P Value | A-? | |
|---|---|---|---|---|---|---|---|
| | | | | | | | |
| Ctrl 3w vs. AB1 3w | 29.3 | 20.99 to 37.62 | Yes | **** | 0.0001 | B | AB1 3w |
| Ctrl 3w vs. AB3 3w | 29.92 | 21.6 to 38.23 | Yes | **** | 0.0001 | C | AB3 3w |

This study shows that IgY can be injected into salmon for slow release of antigen from the injection site and with prolonged preventive effect towards sea lice infection.

### Example 5

The same fish (AB1 and AB3) as described in Example 4, were given a second intraperitoneal injection of a water solution of IgY at 3 weeks post initial challenge with copepodids. Each fish were injected with at a dose of 0.05 mg IgY/g live weight. The average number of sea lice in the two groups AB1 and AB3 were 26.2 and 26.3, respectively, at the time of injection, while the control fish had 51.5 sea lice (average). The controls were left untreated. At 1 and 2 weeks later (4 and 5 weeks post initial challenge) the number of sea lice was counted in individual fish, including the controls.

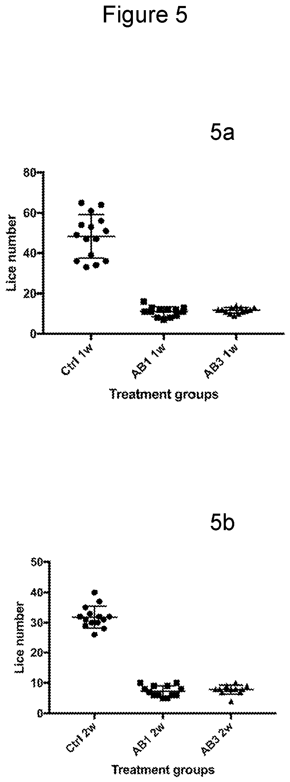

One week after injection of water soluble IgY, the number of sea lice had dropped to 11 and 11.7 in groups AB1 and AB3, respectively, as shown in figure 5a. The number of sea lice in the controls were at 48.3, representing a reduction in number of sea lice of 76% and 75%, for AB1 and AB3, respectively. 2 weeks after injection, the overall sea lice numbers in the tank dropped and controls are at 32 lice/fish while the IgY injected fish are at 7.3 and 7.8 for the AB1 and AB3, respectively, as shown in figure 5b.

This example shows that salmon infected with sea lice can be treated against sea lice infection by injection of IgY at a dose of 0.05 mg/g of live weight of the fish with a marked reduction in number of sea lice as a result of treatment. The treatment adds an extra 25% reduction on top of the 50% reduction achieved through injection of an oil-formulated IgY emulsion.

## Claims

1. IgY antibody composition obtained by immunization of an egg-laying poultry with any development stage of a Caligidae sea louse.

2. IgY antibody composition according to claim 1, wherein the Caligidae is a Lepeophtheirus, preferably *Lepeophtheirus salmonis.*

3. IgY antibody composition according to claim 1, wherein the Caligidae is a Caligus, preferably Caligus rogercresseyi.

4. IgY antibody composition according to any of the claims 13, wherein the egg-laying poultry is;
a) immunized with a Caligidae,
b) eggs provided by the immunized poultry are collected,
c) preparation of the IgY composition, wherein the egg yolk of the collected eggs is processed, and said IgY is isolated.

5. IgY antibody composition according to any of the preceding claims, wherein the Caligidae comprises one of the development stages, or a combination of two or more of the development stages, or is a mixture of all the different development stages of said Caligidae.

6. IgY composition according to claim 1 for use in reduction of infestation and/or prevention and/or treatment of infection of a Salmonidae by a parasite of the order Caligidae,

7. IgY composition for use according to claim 6 wherein the Caligidae is a Lepeophtheirus, preferably *Lepeophtheirus salmonis.*

8. IgY composition according to claim 6 for use in reduction of infestation and/or prevention and/or treatment of infection of a Salmonidae by a parasite of the order Caligidae, wherein the Caligidae is a Caligus, Caligus rogercresseyi.

9. IgY composition according to claim 6 for use in reduction of infestation and/or prevention and/or treatment of infection of a Salmonidae by a parasite of the order Caligidae, wherein the administration route is oral, preferably wherein the IgY composition is administered as a component of the feed given to the Salmonidae.

10. IgY composition according to claim 6 for use in reduction of infestation and/or prevention and/or treatment of infection of a Salmonidae by a parasite of the order Caligidae, wherein the administration route is by injection, preferably intraperitoneally, preferably as watery solution or any formulation for retaining said IgY at the injection site providing slow release.

11. IgY composition according to claim 6 for use in reduction of infestation and/or prevention and/or treatment of infection of a Salmonidae by a parasite of the order Caligidae, wherein the Salmonidae is infected by a Caligidae of the genera Lepeophtheirus or Caligus.

12. IgY composition according to claim 6 for use in reduction of infestation and/or prevention and/or treatment of infection of a Salmonidae by a parasite of the order Caligidae, wherein the Salmonidae is infected by *Lepeophtheirus salmonis* or *Caligus rogercresseyi.*

13. IgY composition according to claim 6 for use in reduction of infestation and/or prevention and/or treatment of infection of a Salmonidae by a parasite of the order Caligidae, wherein said Salmonidae is selected from the group consisting of salmon, charr and trout.

14. A pharmaceutical composition comprising an IgY antibody composition according to any of the claims 1-5, and at least one medically compatible adjuvant.

15. A vaccine composition comprising an IgY antibody composition according to any of the claims 1-5.

## Patentansprüche

1. IgY-Antikörper-Zusammensetzung, erhalten durch Immunisierung eines eierlegenden Geflügels mit einem beliebigen Entwicklungsstadium einer Caligidae-Seelaus.

2. IgY-Antikörper-Zusammensetzung nach Anspruch 1, wobei die Caligidae ein Lepeophtheirus ist, vorzugsweise *Lepeophtheirus salmonis.*

3. IgY-Antikörper-Zusammensetzung nach Anspruch 1, wobei die Caligidae ein Caligus ist, vorzugsweise Caligus rogercresseyi.

4. IgY-Antikörper-Zusammensetzung nach einem der Ansprüche 1 3, wobei das eierlegende Geflügel Folgendes ist;
a) mit einem Caligidae immunisiert,
b) vom immunisierten Geflügel bereitgestellte Eier werden gesammelt,
c) Herstellung der IgY-Zusammensetzung, wobei das Eigelb der gesammelten Eier verarbeitet wird und das IgY isoliert wird.

5. IgY-Antikörper-Zusammensetzung nach einem der vorhergehenden Ansprüche, wobei die Caligidae eines der Entwicklungsstadien oder eine Kombination von zwei oder mehr der Entwicklungsstadien umfasst oder ein Gemisch von allen der verschiedenen Entwicklungsstadien der Caligidae ist.

6. IgY-Zusammensetzung nach Anspruch 1 zur Verwendung bei einer Verringerung eines Befalls und/oder einer Vorbeugung und/oder einer Behandlung einer Infektion einer Salmonidae durch einen Parasiten der Ordnung Caligidae.

7. IgY-Zusammensetzung zur Verwendung nach Anspruch 6, wobei die Caligidae ein Lepeophtheirus ist, vorzugsweise *Lepeophtheirus salmonis.*

8. IgY-Zusammensetzung nach Anspruch 6 zur Verwendung bei einer Verringerung eines Befalls und/oder einer Vorbeugung und/oder einer Behandlung einer Infektion einer Salmonidae durch einen Parasiten der Ordnung Caligidae, wobei die Caligidae ein Caligus, Caligus rogercresseyi, ist.

9. IgY-Zusammensetzung nach Anspruch 6 zur Verwendung bei einer Verringerung eines Befalls und/oder einer Vorbeugung und/oder einer Behandlung einer Infektion einer Salmonidae durch einen Parasiten der Ordnung Caligidae, wobei die Art der Verabreichung oral ist, wobei vorzugsweise die IgY-Zusammensetzung als ein Bestandteil des Futters, das der Salmonidae gegeben wird, verabreicht wird.

10. IgY-Zusammensetzung nach Anspruch 6 zur Verwendung bei einer Verringerung eines Befalls und/oder einer Vorbeugung und/oder einer Behandlung einer Infektion einer Salmonidae durch einen Parasiten der Ordnung Caligidae, wobei die Art der Verabreichung durch Injektion ist, vorzugsweise intraperitoneal, vorzugsweise als wässrige Lösung oder eine beliebige Formulierung zum Zurückhalten des IgY an der Injektionsstelle, die eine langsame Freisetzung bereitstellt.

11. IgY-Zusammensetzung nach Anspruch 6 zur Verwendung bei einer Verringerung eines Befalls und/oder einer Vorbeugung und/oder einer Behandlung einer Infektion einer Salmonidae durch einen Parasiten der Ordnung Caligidae, wobei die Salmonidae durch eine Caligidae der Gattungen Lepeophtheirus oder Caligus infiziert ist.

12. IgY-Zusammensetzung nach Anspruch 6 zur Verwendung bei einer Verringerung eines Befalls und/oder einer Vorbeugung und/oder einer Behandlung einer Infektion einer Salmonidae durch einen Parasiten der Ordnung Caligidae, wobei die Salmonidae durch *Lepeophtheirus salmonis* oder *Caligus rogercresseyi* infiziert ist.

13. IgY-Zusammensetzung nach Anspruch 6 zur Verwendung bei einer Verringerung eines Befalls und/oder einer Vorbeugung und/oder einer Behandlung einer Infektion einer Salmonidae durch einen Parasiten der Ordnung Caligidae, wobei die Salmonidae aus der Gruppe bestehend aus Lachs, Saibling und Forelle.

14. Pharmazeutische Zusammensetzung, umfassend eine IgY-Antikörper-Zusammensetzung nach einem der Ansprüche 1 - 5 und mindestens ein medizinisch verträgliches Adjuvans.

15. Impfstoffzusammensetzung, umfassend eine IgY-Antikörper-Zusammensetzung nach einem der Ansprüche 1 - 5.

## Revendications

1. Composition d'anticorps IgY obtenue par immunisation d'une volaille pondeuse avec n'importe quel stade de développement d'un pou de mer Caligidae.

2. Composition d'anticorps IgY selon la revendication 1, ledit Caligidae étant un Lepeophtheirus, de préférence *Lepeophtheirus salmonis.*

3. Composition d'anticorps IgY selon la revendication 1, ledit Caligidae étant un Caligus, de préférence Caligus rogercresseyi.

4. Composition d'anticorps IgY selon l'une quelconque des revendications 1-3, ladite volaille pondeuse étant ;
a) immunisée avec un Caligidae,
b) les œufs fournis par la volaille immunisée étant collectés,
c) la préparation de la composition d'IgY, le jaune d'œuf des œufs collectés étant traité, et ladite IgY étant isolée.

5. Composition d'anticorps IgY selon l'une quelconque des revendications précédentes, ledit Caligidae comprenant l'un des stades de développement, ou une combinaison de deux stades de développement ou plus, ou étant un mélange de tous les différents stades de développement dudit Caligidae.

6. Composition d'IgY selon la revendication 1 pour utilisation dans la réduction de l'infestation et/ou la prévention et/ou le traitement de l'infection d'un salmonidé par un parasite de l'ordre des Caligidae.

7. Composition d'IgY pour utilisation selon la revendication 6, ledit Caligidae étant un Lepeophtheirus, de préférence *Lepeophtheirus salmonis.*

8. Composition d'IgY selon la revendication 6 pour utilisation dans la réduction de l'infestation et/ou la prévention et/ou le traitement de l'infection d'un salmonidé par un parasite de l'ordre des Caligidae, ledit Caligidae étant un Caligus, Caligus rogercresseyi.

9. Composition d'IgY selon la revendication 6 pour utilisation dans la réduction de l'infestation et/ou la prévention et/ou le traitement de l'infection d'un salmonidé par un parasite de l'ordre des Caligidae, la voie d'administration étant orale, de préférence ladite composition d'IgY étant administrée sous forme de composant de la nourriture donnée aux salmonidés.

10. Composition d'IgY selon la revendication 6 pour utilisation dans la réduction de l'infestation et/ou la prévention et/ou le traitement de l'infection d'un salmonidé par un parasite de l'ordre des Caligidae, ladite voie d'administration étant par injection, de préférence par voie intrapéritonéale, de préférence sous forme de solution aqueuse ou toute formulation permettant de retenir ladite IgY au niveau du site d'injection qui assure une libération lente.

11. Composition d'IgY selon la revendication 6 pour utilisation dans la réduction de l'infestation et/ou la prévention et/ou le traitement de l'infection d'un salmonidé par un parasite de l'ordre des Caligidae, ledit salmonidé étant infecté par un Caligidae des genres Lepeophtheirus ou Caligus.

12. Composition d'IgY selon la revendication 6 pour utilisation dans la réduction de l'infestation et/ou la prévention et/ou le traitement de l'infection d'un salmonidé par un parasite de l'ordre des Caligidae, ledit salmonidé étant infecté par *Lepeophtheirus salmonis* ou *Caligus rogercresseyi.*

13. Composition d'IgY selon la revendication 6 pour utilisation dans la réduction de l'infestation et/ou la prévention et/ou le traitement de l'infection d'un salmonidé par un parasite de l'ordre des Caligidae, ledit salmonidé étant choisi dans le groupe constitué par le saumon, l'omble chevalier et la truite.

14. Composition pharmaceutique comprenant une composition d'anticorps IgY selon l'une quelconque des revendications 1 à 5, et au moins un adjuvant médicalement compatible.

15. Composition de vaccin comprenant une composition d'anticorps IgY selon l'une quelconque des revendications 1 à 5.
